# EUROPEAN PATENT APPLICATION

(11) **EP 1 762 624 A2**
(43) Date of publication of application: **14.03.2007**
(21) Application number: 06010492.4
(22) Date of filing: 21.12.2000
(51) Int. Cl.: C12N 15/85, A01K 67/027

(54) **Methods for amplifying genetic material and uses thereof**

(30) Priority: 22.01.2000 GB 0001401
(62) Divisional of application: 00985674.1
(71) Applicant: THE UNIVERSITY COURT OF THE UNIVERSITY OF EDINBURGH, Edinburgh EH8 9YL (GB)
(72) Inventor: Melton, David William, Edinburgh EH10 7AD (GB)
(74) Representative: Chapman, Paul Gilmour

(57) **Abstract**

The present invention concerns a method of making a transgenic non-human animal cell which is totipotent or totipotent for nuclear transfer and which cell comprises amplified copies of a nucleic acid sequence of interest. The method comprising subjecting a cell from a host cell population to a gene amplification protocol to produce a cell which retains its totipotency or totipotency for nuclear transfer and which comprises amplified copies of the nucleic acid sequence of interest. Because the resulting cells retain totipotency, or totipotency for nuclear transfer, they may be used to generate a transgenic non-human animal which expresses amplified copies of a gene of interest. Consequently, high levels of a product of interest may be obtained from the animal.

## Description

This invention relates to methods for amplifying genetic material and to the use of such methods for the generation of transgenic non-human animals having amplified copies of a nucleic acid sequence of interest and from which high levels of a product of interest may be obtained. The invention also relates to methods for achieving, in a single-step, the amplification of a nucleic acid sequence of interest in cultured eukaryotic cells.

The discovery, of methods for introducing DNA into living cells in a functional form has provided the opportunity for creating transgenic cells which express commercially significant proteinaceous products. Prokaryotic cells and the simpler eukaryotes, such as yeasts, have been traditionally employed in these methods. Animal cells are now increasingly being used, especially where the transgene is animal-derived. Animals cells offer several advantages as hosts for transgenic animal DNA, and the more similar the animal species from which the gene is derived, the greater the likelihood that a functional product will be expressed and processed correctly. Different animal species frequently produce analogous proteins, and often considerable genetic homology is carried over from one species to another. Accordingly, an animal cell is much more likely than a bacterial or yeast cell to be able to perform the post-translational processing steps necessary for the gene-coded protein to be biologically active and is much more likely to translate a foreign gene having interrupted coding sequences correctly.

Techniques have been developed for introducing foreign genetic material into the genomes of animal tissue cells. These include attaching the foreign genetic material to a cloning vector, such as a modified virus or cosmid, and transforming the cloning vector into the cell. The genetic material introduced into the cell by means of the vector will frequently incorporate into the genome.

An obstacle to using animal tissue cell cultures, particularly those obtained from higher animals, for the expression of recombinant gene product is the general unsuitability of tissue cell culture to large scale production processes. Unlike bacteria or yeast cells, which rapidly proliferate in a favourable environment, animal tissue cells are much less amenable to mass production techniques. The cells which comprise the tissues of higher animals reproduce slowly and in many cases, having reached a mature stage, do not reproduce at all. The division of animal tissue cells is often very much influenced by the environs of other cells. Even if a tissue culture is initially provided with a generally ideal environment, the proliferation of cells changes that environment, frequently unfavourably to further proliferation. Tissue cultures are also subject to infection, and a major infection could wipe out a considerable investment of time and effort.

The advent of transgenic techniques which enable the production of transgenic animals provides the possibility of obtaining correctly processed animal gene products in substantially greater quantities than has been possible with tissue cultures. A living transgenic animal and its progeny can potentially provide a continuous source of a transgenic product.

Conventionally, transgenic animals are produced by injection of transgene DNA into the pronucleus of a fertilised egg. Attempts to maximise the yield of a transgene product of interest have concentrated on maximising expression of individual genes (26, 27).

However, we have reasoned that a gene amplification system that could be applied to the animal cell types that can be used to produce transgenic animals, would offer the prospect of increased yield of gene products of interest.

Amplification of chromosomal genes has been practised widely in cultured mammalian cells and a variety of procedures have been devised to select for the amplification of specific chromosomal genes (1).

We have reasoned that gene amplification can be exploited to increase the yield of gene products of interest in cultured animal cells. Typically, in conventional gene transfer, a nucleic acid sequence of interest is cotransformed into an animal cell with a selectable marker gene. Cells with small numbers of cointegrated copies of both genes, and which express both the selectable marker and the nucleic acid sequence of interest, are obtained.

By cotransforming into a cultured animal cell, a gene which can be selected for gene amplification, along with a non-selectable nucleic acid sequence of interest to be expressed in the same cell, gene amplification can be exploited to produce much larger amounts of any particular gene product than through conventional gene transfer alone. This is because application of selective pressure for the amplification of the selectable gene often results in the coamplification of the second, non-selectable gene, leading to the synthesis of large amounts of the product of interest.

Gene amplification protocols in cultured animal cells may utilise the dihydrofolate reductase (DHFR) gene and the use of DHFR inhibitors, such as methotrexate. Normal cells, with one or two copies of the DHFR gene, cannot survive in low concentrations of methotrexate. This provides selective pressure for cells with amplified copies of the DHFR gene, which evade the effect of the inhibitor by producing elevated amounts of DHFR. By progressively increasing the inhibitor concentration, in a series of selective steps, cells with thousands of copies of the amplified DHFR gene can be obtained. This is an efficient amplification system, which can be used to coamplify a gene introduced into the cell along with the DHFR gene and this method may thus be used to produce large amounts of a product of interest.

The DHFR gene amplification system suffers from a number of disadvantages: it requires multiple rounds of selection to achieve the highest amplified gene copy numbers; high concentrations of toxic drugs, such as methotrexate, are required; and the purification of the product of interest can be complicated by the high levels of dihydrofolate reductase that are also present. Some of these disadvantages have been addressed by other amplification systems, such as that involving glutamine synthetase (2).

Several alternative methods have been described for the selection of cotransformed host cells. One strategy involves cotransforming host cells with a nucleic acid sequence of interest and a DHFR gene, and selecting those cells that express high levels of DHFR by directly culturing the cells in a medium containing a high concentration of methotrexate. Many of the cells selected also express the cotransformed product gene at high levels (18).

Another method involves the use of polycistronic mRNA expression vectors containing a nucleic acid sequence of interest at the 5' end of the transcribed region and a selectable gene at the 3' end. Because translation of the selectable gene at the 3' end of the polycistronic mRNA is inefficient, such vectors exhibit preferential translation of the desired product gene and require high levels of polycistronic mRNA to survive selection (19, 20, 21). Accordingly, cells expressing high levels of the desired product (protein) may be obtained by culturing the initial transformants in a medium containing a selective agent appropriate for use with the particular selectable gene.

Unfortunately, these methods suffer from certain drawbacks which limit their usefulness. High expressing cells obtained by direct culturing in medium containing a high level of a selective agent may have poor growth and stability characteristics, thus limiting their usefulness for long-term production processes (18). Such selection for high-level resistance to methotrexate may produce cells with an altered, methotrexate-resistant DHFR enzyme, or cells that have altered methotrexate transport properties rather than cells containing amplified genes (Haber, et al, J. Biol. Chem. 256: 9501 (1981); Assaraf & Schimke, Proc. Nat. Acad. Sci. 84:7154 (1987)). With polycistronic vectors, there is a strong selection for deletion or rearrangement of the 5' nucleic acid sequence of interest when selecting for expression of the 3' selectable gene. Cells harbouring vectors having such mutations in the 5' product gene tend to outgrow other cells in the population, and thus interfere with the selection of cells actually expressing high levels of the nucleic acid sequence of interest (20).

Other selection procedures include those disclosed in WO 83/03259 and WO 92/17566. Briefly, the selection procedure described in WO 83/03259 comprises cotransforming a eukaryotic cell with a nucleic acid sequence of interest and a functionally deficient gene coding for a selectable or identifiable trait, such as adenine phosphoribosyltransferase or xanthine phosphoribosyltransferase activity. Cells which express the nucleic acid sequence of interest are recovered and the cells which express the selectable or identifiable trait are then selected. Cells which express the selectable or identifiable trait represent rare variant cell subclones which have greatly amplified the expression of the functionally deficient gene so that expression occurs at levels characteristic of the normal gene. These rare subclones also contain multiple copies of the nucleic acid sequence of interest.

WO 92/17566 describes the cotransformation of a host cell with a nucleic acid sequence of interest and a selectable gene which is modified by inserting into its transcribed region an intron of such length that the intron is correctly spliced from the corresponding mRNA precursor at lower efficiency. As a consequence thereof, the amount of selectable gene product produced from the intron-modified selectable gene is substantially less than that produced from the starting selectable gene. Following cotransformation of the cells, it is observed that most of the resulting transformants fail to exhibit the selectable phenotype that is characteristic of the selectable gene product, as a consequence of the relatively low level of selectable gene product that is produced from the intron-modified selectable gene in the transformants. A small proportion of the transformants do exhibit the selectable phenotype, and among those transformants, the majority are found to express high levels of the desired protein encoded by nucleic acid sequence of interest.

To date, none of these gene amplification systems have been used nor suggested for use in specialised cultured animal cells, such as embryonic stem (ES) cells. Further, it has been reported that gene amplification is only possible in transformed or p53-deficient cells (eg L R Livingstone et al. (1992) Cell 70:923-35.). However, such reports are clearly disproved by our experiments in which we demonstrate that gene amplification is possible in ES cells. Furthermore, we have found that the ES cells retain totipotency and can be used to generate transgenic *

The present invention provides the use of gene amplification methods for the production of transgenic animals containing amplified copies of a nucleic acid sequence of interest, the transgenic animals having potential to synthesise large amounts of a product of interest.

A first aspect of the present invention provides a method of making a transgenic non-human animal cell which is totipotent or totipotent for nuclear transfer and which cell comprises amplified copies of a nucleic acid sequence of interest. The method comprises subjecting a cell from a host cell population to a gene amplification protocol to produce a cell which retains its totipotency or totipotency for nuclear transfer and which comprises amplified copies of the nucleic acid sequence of interest.

By a "host cell population" we include one or more animal cells. We also include the progeny thereof. The host cell population comprises totipotent cells or cells which are totipotent for nuclear transfer.

By a "nucleic acid sequence of interest" we include a DNA that encodes a desired protein or mRNA product. A nucleic acid sequence of interest may encode an antibody, a viral antigen, an interferon, a growth hormone, insulin, a clotting factor, human serum albumin, an enzyme, or an antisense mRNA.

Preferably, the gene amplification protocol comprises transforming cells of the host cell population with the nucleic acid sequence of interest.

In a preferred embodiment of the first aspect of the invention the gene amplification protocol comprises:
(a) cotransforming cells of the host cell population which are totipotent or which are totipotent for nuclear transfer with the nucleic acid sequence of interest and a partially-disabled selectable marker gene encoding a selectable gene product ; and
(b) culturing cells of the host cell population under conditions which permit selection of cells expressing the selectable gene product at significantly higher levels than cells with substantially no expression of the selectable gene product.

Various methods are known in the art for cotransforming genes into animal cells. One of the most commonly used methods involves direct DNA transfer into cells. For example, DNA may be introduced into animal cells by exposing the cells to DNA in the presence of calcium phosphate precipitate or DEAE-dextran (24). Other methods for introducing genes into animal cells include electroporation, microinjection, or infection with recombinant viruses (19, 24, 25).

Preferably, prior to the cotransformation procedure, cells of the host cell population do not comprise or express the selectable marker gene. Suitably, the animal cells do not express the nucleic acid sequence of interest prior to transformation.

By a "selectable marker gene" we include a DNA that encodes a protein necessary for the growth or survival of a host cell under the particular cell culture conditions chosen. Accordingly, a host cell that is transformed with a selectable marker gene and acquires the selectable trait will be capable of growth or survival under selective cell culture conditions wherein a non-transformed host cell is not capable of growth or survival. Typically, a selectable marker gene will confer resistance to a drug or compensate for a metabolic or catabolic defect in the host cell.

Examples of selectable marker genes are well known in the art. For example, selectable genes commonly used with eukaryotic cells inlcude the genes for aminoglycoside phosphotransferase (APH or neo), hygromycin phosphotransferase (hyg), dihydrofolate reductase (DHFR) and thymidine kinase (tk). However, it should be appreciated that some selectable marker genes are generally used in combination with a chemical that is toxic to the cells and which may damage the totipotency of the cells. Accordingly, it is preferred that selectable marker genes are used which do not necessitate the use of toxic chemicals to achieve selection of the desired cells.

In a preferred embodiment of the present invention the selectable marker gene encodes a purine phosphoribosyltransferase. Preferably, the purine phosphoribosyltransferase is any one of the following: hypoxanthine phosphoribosyltransferase (HPRT), adenine phosphoribosyltransferase (APRT), guanine phosphoribosyltransferase (GPRT) or xanthine phosphoribosyltransferase (XPRT). Preferably, the purine phosphoribosyltransferase is HPRT. Preferably, the HPRT is a eukaryotic HPRT, preferably a mammalian HPRT, more preferably a rodent HPRT, and yet more preferably a murine HPRT.

The term "selectable marker gene" may include a product which confers the host with a visually-detectable phenotype. However, use of a reporter gene in a method of the present invention would necessitate the physical separation of cells which would be tedious and inefficient. Thus in most circumstances it would not be desirable to use a reporter gene as the selectable marker gene.

Selectable marker genes and genes of interest may be obtained from genomic DNA, cDNA transcribed from cellular RNA, or by *in vitro* synthesis.

By a "selectable gene product" we include a protein encoded by a selectable marker gene.

It will be appreciated that cells which comprise only one or two copies of a selectable marker gene may not produce sufficient quantities of the selectable gene product to survive selective culture conditions. Accordingly, it follows that cells with substantially no expression of a selectable gene product may be used successfully in some amplification protocols providing, of course, that the untransformed cells express insufficient quantities of the selectable gene product to survive in the selective medium.

Although amplification of the native gene is a possibility, amplification of an introduced gene is far more likely, because introduced sequences tend to be more labile than native ones. Notwithstanding this, it is preferred that the animal cells do not comprise a native copy of the selectable marker gene. Further, some amplification protocols may not work efficiently, or may not work at all, if the animal cell comprises a native copy of the selectable marker gene.

Accordingly, it is preferred that the cells of the host cell population do not comprise a functional or partially-disabled native copy of the selectable marker gene. Thus prior to transformation with the selectable marker gene, the cells do not comprise a functional or partially-disabled native copy of the selectable marker gene.

The level of expression of a nucleic acid sequence of interest or a selectable marker gene in a host cell may be determined by various methods known in the art. For example, mRNA transcribed from a gene can be quantified by northern hybridisation (22). Protein encoded by a gene can be quantified either by assaying for the biological activity of the protein or by employing assays that are independent of such activity, such as western blotting or radioimmunoassay using antibodies that are capable of reacting with the protein (23).

By a "partially-disabled selectable marker gene" or a selectable marker gene having a degree of "expression disability" we include a gene which is not fully functional, such that its activity is diminished but not eliminated. Accordingly, the gene may encode lower amounts of a selectable gene product and/or it may encode a selectable gene product of lower activity or stability than that produced by a fully functional version of the gene. A partially-disabled selectable marker gene may be produced by mutating a fully functional version of the gene.

The cotransformed host cells are cultured under conditions that permit the selection of cells expressing significantly increased levels of the selectable gene product. By "cells expressing significantly increased levels of the selectable gene product" we include cells which express the selectable gene product at a measurably higher level than an untransformed host cell.

Conveniently, the cells are cultured in a medium which favours growth of cells expressing significantly increased levels of the selectable gene product and disfavours growth of cells with little or no expression of the selectable gene product. Suitable nutrient media and cell culture methods are well known in the art. See, for example, Sherman (1991), Meth. Enzymol. 194:3; Mather (1990) Meth. Enzymol. 185:567; Berlin & Bode (1987), in Basic Biotechnology pp.133-177 (VCH Publishers).

Preferably, the gene amplification protocol requires, or comprises, only one round of selection (ie a single selective step). It will be appreciated that a single selective step has the advantage that the cells need only be cultured for a short time, in comparison with protocols which require multiple rounds of selection. In general the longer a cell is in culture the greater is the probability that it will suffer a genetic or epigenetic change resulting in a loss of totipotency.

Preferably, the partially-disabled selectable marker gene is selected from one of a plurality wherein the genes within the plurality possess differing degrees of expression disability. Suitably, the partially-disabled selectable marker gene is selected with regard to the nature of the host cell population and the desired degree of gene amplification.

Those skilled in the art will appreciate that different cell-types may have different sensitivities to a selective agent. Cells may therefore require different amounts of a selectable gene product to survive a given set of selective conditions, and accordingly the degree of disablement of a selectable marker gene may be chosen to reflect this requirement. For example, mouse embryonic stem cells have a more fastidious requirement for hypoxanthine phosphoribosyltransferase (HPRT) activity than Chinese hamster lung fibroblasts and for this reason it is appropriate to use a HPRT gene having only a small degree of disablement in ES cells, but a higher degree of disablement in fibroblasts.

It will also be appreciated that where a high degree of gene amplification is required it is appropriate to use a selectable marker gene having a high degree of disablement, whereas if only a small amount of gene amplification is required it may be appropriate to use a selectable marker gene having only a small degree of disablement.

Preferably, some or all of the partial-disablement of the selectable marker gene results from one or more of the following:
(a) lack of a transcriptional control sequence normally present in the promoter region, or in an intron;
(b) lack of an intron, or other mRNA processing signal;
(c) a missense mutation, or a nonsense mutation;
(d) insertion of an intron.

Preferably, the partially-disabled selectable marker gene is a murine HPRT minigene with one or more of the following characteristics:
(a) a missense mutation, or a nonsense mutation in a HPRT coding region;
(b) a truncated intron 1 (with the key control element present);
(c) a truncated intron 2;
(d) a truncated or absent intron 7 or 8.

It will be appreciated that murine HPRT may be used in non-murine transformation systems. Indeed where the expression requirements for human and rodent HPRT have been investigated they have been found to be very similar. See Jiralerspong S, Patel PI (1996) Proc Soc Exp Biol Med 212:116-27; and Reid et al (1990) Proc Natl Acad Sci USA 87:4299-303.

By "the key control element" of intron 1, we refer to the element in intron 1 which acts in a position- and orientation-dependent manner to stimulate transcription from a linked promoter.

Preferably, the murine HPRT minigene has a missense mutation of Asp (GAT) 200 - Asn.

Suitably the method of the first aspect of the present invention utilises a plasmid comprising a partially-disabled selectable marker gene.

In a preferred embodiment of the present invention the host cell population comprises totipotent cells, preferably non-human embryonic stem (ES) cells. In another embodiment of the present invention the host cell population comprises cells totipotent for nuclear transfer, preferably cells derived from a non-human embryo, foetus or adult tissue.

A second aspect of the present invention provides a transgenic non-human animal cell which is totipotent or totipotent for nuclear transfer and which comprises amplified copies of a nucleic acid sequence of interest, which cell is obtainable by a method of the first aspect of the invention.

A third aspect of the present invention provides a method of making a transgenic non-human animal which expresses multiple copies of a nucleic acid sequence of interest and which thereby produces a useful level of a product of interest, which method comprises employing a cell according to the second aspect of the invention, or the genetic material thereof, to generate a transgenic non-human animal.

By a "product of interest" we include a product encoded by a nucleic acid sequence of interest. Usually, the product will be a protein.

Various methods for creating transgenic animals are known in the art. The principal means by which transgenic animals are currently produced are: pronuclear DNA microinjection; blastocyst microinjection or morula aggregation of embryonic stem (ES) cells; and replication-defective viral vector transduction (17).

The method chosen for producing a particular transgenic animal will be influenced by the relative merits and limitations of the different methods and the host cells used in the gene amplification procedure. In one preferred embodiment the host cell population comprises totipotent cells, preferably non-human embryonic stem (ES) cells. In another preferred embodiment the host cell population comprises cells totipotent for nuclear transfer, preferably cells derived from a non-human embryo, foetus or adult tissue.

ES cells may be used to produce a transgenic animal containing coamplified copies of the nucleic acid sequence of interest by established procedures (9). Briefly, chimaeric animals are produced, either by injecting ES cells into host blastocysts, or by aggregating ES cells with host morulae. In each case, the chimaeric embryos are reimplanted into foster mothers and allowed to develop into chimaeric animals. If the ES cells have contributed to the germ line of the chimaera, then some gametes from the chimaera will be ES cell-derived. By crossing a chimaera with another animal, progeny with ES cell-derived genetic material can be obtained. If the ES cells used contain coamplified copies of the nucleic acid sequence of interest, some of the progeny will contain the coamplified gene in all its somatic cells. In this way transgenic strains containing the coamplified gene can be established.

While the ES system has been developed in the mouse other animal species (for instance mammalian species) may be used for the purposes of this invention. Isolation of ES cells is described in Robertson 1987 (9).

A second method of producing transgenic animals, which is likely to be particularly valuable in larger mammalian species, such as sheep and cattle may also be used to generate a transgenic animal of the present invention. The basic procedure has been described for the cloning of sheep (10, 11).

Briefly, a cell line was established from a day 9 sheep embryonic disc. Nuclear transfer from these cells into enucleated oocytes resulted in the production of viable lambs. The procedure was subsequently repeated using nuclei from foetal fibroblasts and, in one case, from adult mammary epithelial cell cultures. Isolation of cells with little or no expression of a given selectable gene product from derivatives of such cell lines, derived from early animal embryos, foetuses, or adult tissues and which retain totipotency for nuclear transfer, will permit use of the gene amplification procedure described herein and the production, by nuclear transfer into enucleated oocytes, of transgenic animals containing coamplified copies of a nucleic acid sequence of interest.

See also, Schnieke AE, et al. (1997) Science 278:2130-3.WO 97/07669, WO 98/30683 and Sims et al. (1993), Proc. Natl. Acad. Sci. USA 90:6143-6147 for further information on the production of transgenic animals using nuclear transfer protocols.

A fourth aspect of the present invention provides a transgenic non-human animal which is totipotent or totipotent for nuclear transfer and which comprises amplified copies of a nucleic acid sequence of interest, which animal is obtainable by a method of the third aspect of the present invention.

A transgenic animal of the present invention may be a chimaera or it may express multiple copies of a nucleic acid sequence of interest in all its somatic cells. Also, a transgenic animal of the present invention may be a first generation transgenic animal or any of its progeny which comprise multiple copies of the nucleic acid sequence of interest.

Preferably, a transgenic animal of the present invention expresses substantial amounts of the product of interest, either constitutively or in a regulated manner, throughout the entire body or restricted to a particular tissue or body fluid.

Methods for achieving the tissue-specific expression of a transgene are amply described in the art. For example, the metallothionein promoter has been used to direct the expression of the rat growth hormone in the liver tissue of transgenic mice (Palmiter et al (1982), Nature 300:611). Another example is the elastase promoter, which has been shown to direct the expression of foreign genes in the pancreas (Ornitz et al (1985), Nature 313:600). See also EP 279 582, which describes methods for the targeting of proteins to the mammary gland and the subsequent secretion of biologically important molecules in the milk.

Developmental control of gene expression has also been achieved in transgenic animals, i.e. the foreign gene is transcribed only during a certain time period, and only in a certain tissue. For example, Magram et al (1985 Nature 315:338) demonstrate the developmental control of genes under the direction of a globin promoter.

Proteins produced by a transgenic animal of the present invention may then be harvested e.g. from its serum, milk or ascites fluid. The desired protein may then be purified from other host proteins by methods well known in the art to obtain preparations of the desired protein that are substantially homogeneous.

While the techniques described herein may be used to generate transgenic animals which produce commercially important proteins, it is also envisaged that the methods of the invention may be used to generate animals having improved performance, e.g. in growth rate or meat quality. Animals of the present invention may also be useful in research. Areas of possible research include cancers arising from gene amplification, the consequences of overproducing growth factors, receptors, or other .signalling molecules in development and to combat disease.

In principle, the present invention is potentially applicable to all animals, including birds, such as domestic fowl, amphibian species and fish species. In practice, however, it will be to non-human animals, especially (non-human) mammals, particularly placental mammals, that the greatest commercial utility is presently envisaged. It is with ungulates, particularly economically important ungulates such as cattle, sheep, and pigs that the invention is likely to be particularly useful. It should also be noted that the invention is also applicable to other economically important animal species such as, for example, rodents e.g. rats or mice, or rabbits and guinea pigs.

The invention presented herein is based on the ability, in a specialised set of circumstances, to select in a single step, for the large scale amplification of the hypoxanthine phosphoribosyltransferase (HPRT) gene. This gene codes for an enzyme (hypoxanthine phosphoribosyltransferase; EC 2.4.2.8; alternatively described as hypoxanthine-guanine phosphoribosyltransferase, HGPRT) involved in the purine salvage pathway in mammalian cells, where it phosphoribosylates either of the purine bases hypoxanthine or guanine, to form IMP or GMP respectively. Cells lacking HPRT activity can be easily selected by their ability to grow in the presence of purine analogues, such as 6-thioguanine, which are phosphoribosylated by HPRT to produce toxic products. Conversely, cells with HPRT activity are selected by their ability to grow in HAT medium (4). HAT medium contains: Aminopterin, an inhibitor of dihydrofolate reductase, to block *de novo* purine and pyrimidine synthesis and so make cells dependent on functional salvage pathways; Hypoxanthine, as a substrate for HPRT and the purine salvage pathway; Thymidine, as a substrate for the pyrimidine salvage pathway.

The amplification of the mouse chromosomal HPRT gene has previously been described (5). A 6-thioguanine-resistant derivative (NB-) of a mouse neuroblastoma cell line (NB+) contained an HPRT missense mutation, Asp (GAT) 200 - Asn (AAT). The mutant protein had reduced catalytic ability and poor heat stability. Selection of NB- cells in HAT medium provided selective pressure for the isolation of revertant (NBR) cell lines with up to 50 amplified copies of the chromosomal HPRT gene. NBR cells survive in HAT medium by producing elevated amounts of the mutant protein.

The X-chromosome-linked mouse HPRT gene is 33kb long and contains nine exons. A series of truncated versions of the HPRT gene (minigenes), which are cloned on plasmid vectors and can be introduced into, and expressed efficiently in, cultured mammalian cells have been developed (6). The introduction and expression of a single copy of one of these fully-functional HPRT minigenes in an HPRT-deficient cell is sufficient to confer HAT resistance in a range of cultured cell types (see Example 1 and refs. 7 & 8). The sequence requirements for efficient minigene expression have been investigated (6). In addition to the promoter region, there is a requirement for the aforementioned key control element in intron 1. There is also a non-specific requirement for a number of additional introns in the minigene to achieve maximal expression.

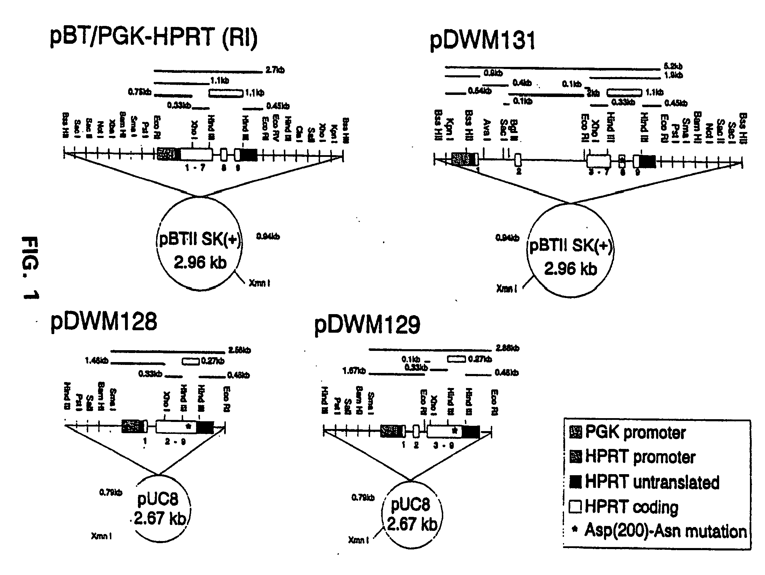

Cells lacking HPRT activity are isolated by their ability to grow in the presence of 6-thioguanine. A 6-thioguanine concentration in the range 5-20 µg/ml is suitable for most mammalian cell types. The use of partially expression-disabled HPRT minigenes permits one-step selection in HAT medium for cells containing up to thousands of copies of both amplified HPRT minigenes and the coamplified nucleic acid sequence of interest, when the nucleic acid sequence of interest is cotransformed, along with a partially-disabled HPRT minigene, into HPRT-deficient cultured mammalian cells. The cells will thus produce large amounts of the product of interest. A series of HPRT minigenes, with varying degrees of expression disability, permit the system to be used in a single selection step, to achieve variable levels of amplification of the HPRT minigene and the cotransformed nucleic acid sequence of interest, in a range of different mammalian cell types. All the partially-disabled HPRT minigenes contain the Asp(200)-Asn missense mutation and so encode an HPRT protein with reduced catalytic activity and poor thermal stability. The vectors vary in the number of introns present (Figure 1). pDWM131 is the least disabled of the series, containing a truncated intron 1 (with the key control element present), a truncated intron 2, and introns 7 and 8. pDWM129 contains the same truncated intron 1 as pDWM131, a more truncated version of intron 2, but lacks introns 7 and 8. pDWM128 contains only the truncated intron 1 and is thus the most disabled minigene in the series. pDWM131 was constructed from pDWM110; pDWM129 was constructed from pDWM127, and pDWM128 was constructed from pDWM126, all by the incorporation of the Asp(200)-Asn missense mutation.

The construction of pDWM110, pDWM127 and pDWM126 has been previously described (6).

### Construction of pDWM131 from pDWM110

The fully functional HPRT minigene pDWM110 was cut with Xhol (site within exon 3) and Ncol (site within intron 8) and the 7.2 kb backbone (Fragment 1) was gel-purified away from the 1 kb fragment running from exon 3 to intron 8. The fully functional HPRT minigene pBT/PGK-HPRT (RI) (ref. 6) was cut with Xhol and ScaI and the 0.6 kb fragment (Fragment 2) running from the Xhol site in exon 3 to the ScaI site in exon 8 was gel-purified. The ScaI site is located upstream of the Asp(200) codon which is mutated Asp(200)-Asn in all the partially-disabled HPRT minigenes. The final fragment (Fragment 3), a 0.4 kb fragment running from the ScaI site in exon 8 to the Ncol site in intron 8 and containing the Asp(200)-Asn missense mutation was obtained by PCR. The template was genomic DNA from the NBR4 cell line (ref 5) which has amplified copies of the chromosomal mouse HPRT gene with the missense mutation. Primers were used to amplify the 3' end of the HPRT gene from NBR4 cells. The forward primer was from exon 8 upstream of the Scal site, the reverse primer was from exon 9. Primers were from mouse HPRT cDNA sequence, GenBank Acc. No. J00423.
exon 8 Forward (5')GTTTGTTGTTGGATATGCCCTTGAC
exon 9 Reverse (5')GCAGATGGCCACAGGACTAGAAC
25 cycles: 94°C 1 min, 67°C 1 min, 72°C 1 min

50µl reaction with 100ng template DNA, 300ng each primer, 2.5 units Taq DNA polymerase in 5 mM KCI/0.15 mM MgCl₂/ 1 mM Tris-HCl/ 0.045% Triton X-100/ 0.045 % Tween 20/ 0.4 mM Na₂ EDTA/ 0.1 mM dNTPs.

The 0.8 kb product was cut with ScaI and NcoI and Fragment 3 was gel-purified. Fragments 1-3 were then ligated together via their unique cohesive ends to generate plasmid pDWM131. The presence of the Asp(200)-Asn missense mutation in pDWM131 and the lack of any other alterations in the region of pDWM131 derived from Fragment 3 was confirmed by DNA sequencing.

### Construction of pDWM128 from pDWM126

pDWM126 was cut with Xhol (site within exon 3) and EcoRI (site at 3' end of minigene) and the 4.2 kb plasmid backbone was gel-purified away from the 1.1 kb fragment running from exon 3 to the 3' end of the minigene. Mouse HPRT cDNA clone pHPT5 (ref. 12), derived from the NBR4 cell line and containing the Asp(200)-Asn missense mutation was cut with PstI (artificial sites at each end of the cDNA insert), the ends were made blunt with Klenow and EcoRI linkers were attached. pHPT5 was then recut with XhoI (site within exon 3) and EcoRI and the 1.1 kb XhoI-EcoRI fragment, running from exon 3 to the 3' end of the cDNA and containing the Asp(200)-Asn missense mutation was gel-purified and ligated into the 4.2 kb plasmid backbone of pDWM126 to give pDWM128.

### Construction of pDWM129 from pDWM127

pDWM127 was cut with Xhol (site within exon 3) and EcoRI (site at 3' end of minigene) and the 4.5 kb plasmid backbone was gel-purified away from the 1.1 kb fragment running from exon 3 to the 3' end of the minigene. Mouse HPRT cDNA clone pHTP5 (ref. 12), derived from the NBR4 cell line and containing the Asp(200)-Asn missense mutation was cut with PstI (artificial sites at each end of the cDNA insert), the ends were made blunt with Klenow and EcoRI linkers were attached. pHPT5 was then recut with Xhol (site within exon 3) and EcoRI and the 1.1 kb XhoI-EcoRI fragment, running from exon 3 to the 3' end of the cDNA and containing the Asp(200)-Asn missense mutation was gel-purified and ligated into the 4.5 kb plasmid backbone of pDWM127 to give pDWM129.

The choice of partially-disabled HPRT minigene used will depend on the degree of gene amplification required and the particular HPRT-deficient cell type to be used. For instance, mouse embryonic stem cells have more fastidious requirements for HPRT expression than Chinese hamster lung fibroblasts (Ref. 6 and Examples 1 and 2) and for this reason the use of the least disabled minigenes in ES cells is likely to be most appropriate. The vector containing the partially-disabled HPRT minigene and the vector containing the gene to be coamplified are both linearized, prior to being introduced into the HPRT-deficient cells. It is not necessary for coamplification that the partially-disabled HPRT minigene and the nucleic acid sequence of interest are present in the same vector DNA molecule. HAT selection for gene amplification is applied 24 hours after gene transfer and HAT-resistant colonies, containing amplified copies of the partially-disabled HPRT minigene and coamplified nucleic acid sequence of interest, can be isolated. The length of time in HAT selection required to isolate colonies with amplified HPRT minigenes will depend on the cell type used.

If the one-step gene amplification protocol has been carried out in HPRT-deficient derivatives of established animal or mammalian cell lines, such as Chinese hamster lung fibroblasts, then clones containing amplified copies of the HPRT minigene and of the coamplified nucleic acid sequence of interest can be expanded and, depending on the nature of the coamplified gene product produced, either cells or culture medium can be harvested to collect the product of interest.

The highly unstable nature of the HPRT protein produced in the HPRT-amplified cell lines should facilitate the purification of the product of interest encoded by the coamplified nucleic acid sequence of interest. It has previously been noted that if cells produce large amounts of two proteins (the protein encoded by the nucleic acid sequence of interest and the protein encoded by the selectable marker) as a consequence of successful amplification, then the biochemical purification of the protein from the nucleic acid sequence of interest will be complicated by the large amount of the other protein present. However, if the protein encoded by the selectable marker is unstable then it will rapidly degrade and not encumber the purification of the protein from the nucleic acid sequence of interest.

If the intention is to produce transgenic animals, with amplified copies of a nucleic acid sequence of interest, then the gene amplification procedure will be carried out, either in HPRT-deficient embryonic stem (ES) cells, or in HPRT-deficient derivatives of primary cultures isolated from embryos, foetuses, or adult tissues. If transgenic animals are to be produced, the vector carrying the partially-disabled HPRT minigene and the vector carrying the nucleic acid sequence of interest to be coamplified should both be cut in such a way that the HPRT minigene and the nucleic acid sequence of interest are released from vector sequences prior to gene transfer. This will reduce the risk of incorporation of vector sequences into the amplified units of HPRT minigene and nucleic acid sequence of interest, which could have an adverse effect on expression.

While this invention has been exemplified using partially-disabled mouse HPRT minigenes, it could operate with any nucleic acid sequence encoding a partially-disabled HPRT activity. Furthermore, by altering the selective medium used, it could also operate with any nucleic acid sequence encoding any partially-disabled purine phosphoribosyltransferase activity, such as adenine phosphoribosyltransferase, guanine phosphoribosyltransferase or xanthine phosphoribosyltransferase. Indeed, the invention also extends to the use of other nucleic acid sequences coding for other marker proteins, provided that suitable selective methods exist to achieve one-step amplification of the marker gene in cultured animal cells and that the selective methods used do not adversely affect the totipotency of the specialised animal cell lines that are used to produce transgenic animals with amplified copies of the nucleic acid sequence of interest.

The methods of the present invention will be useful in the production of many different proteins in the industrial, agricultural, and pharmaceutical fields, and especially useful in the production of proteins that are available in limited quantities from natural sources, including such proteins as growth hormones, interferons, neurotrophic factors, DNase, erythropoietin, inhibin, insulin, relaxin, and tissue plasminogen activator.

The invention is hereinafter described in more detail by way of example only, with reference to the accompanying figures, in which:-

**Figure 1** Structure of HPRT minigenes. The structure of the fully functional HPRT minigene pBT/PGK-HPRT (RI) and the partially-disabled minigenes pDWM128, 129 and 131 are shown schematically. pBT/PGK-HPRT (RI) and pDWM131 are cloned in pBluescript II SK(+), while pDWM128 and 129 are cloned in pUC8. In pBT/PGK-HPRT (RI), the minigene is under the control of the mouse phosphoglycerate kinase gene promoter, while in the three partially-disabled minigenes, the natural mouse HPRT gene promoter is used. The positions, within each minigene, of the nine exon coding blocks from the chromosomal HPRT gene are indicated. The position of the Asp(200) - Asn missense mutation, within exon 8 of the partially-disabled minigenes, is indicated by an asterisk. Note, within pDWM128 and 129, the location of the internal 270bp HindIII restriction fragment, used to determine the level of minigene amplification by Southern blotting. For pBT/PGK-HPRT (RI) and pDWM131, the equivalent part of the coding region, for copy number determination, is located on a 1.1kb internal HindIII fragment.

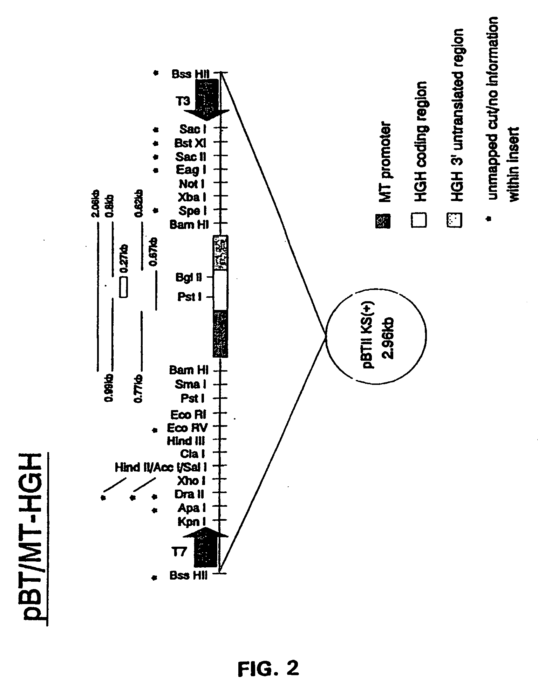

**Figure 2** Structure of human growth hormone plasmid. pBT/MT-HGH is cloned in pBluescript II KS(+). The human HGH coding region is under the control of the mouse MT-I gene promoter, with polyadenylation signals provided by the 3' untranslated region of the human HGH gene. Note the location of the 270bp internal PstI- BglII restriction fragment, used to determine the level of pBT/MT-HGH gene amplification by Southern blotting.

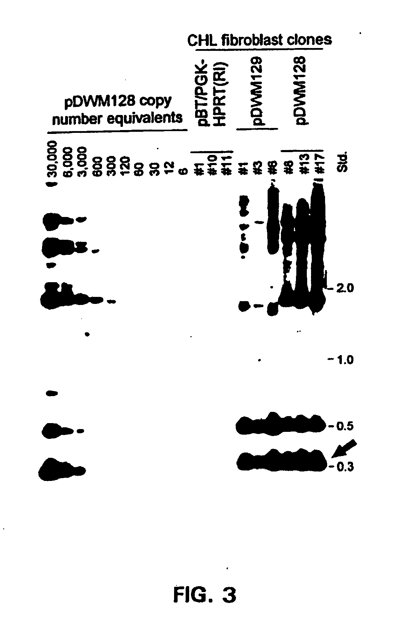

**Figure 3** Determination of HPRT minigene copy number in HAT-resistant RJK88 cells following the one-step gene amplification procedure. Genomic DNA (3 µg) from HAT-resistant Chinese hamster lung (CHL) fibroblast clones, cotransformed with pBT/MT-HGH and pBT/PGK-HPRT(RI), pDWM128 or 129, was restricted with EcoRI/HindIII, electrophoresed and probed with the mouse HPRT cDNA clone pHPT5 (12). In parallel, RJK88 DNA (3 µg) was spiked with varying amounts of pDWM128 plasmid DNA, corresponding to the indicated number of genomic equivalents, restricted with EcoRI/HindIII, electrophoresed on the same gel and probed to provide a calibration curve, against which the HPRT minigene copy number in the HAT-resistant clones could be determined. This was done by comparing the intensity of the indicated 270 bp internal HindIII fragment (see Figure 1). Note that the copy number determined from the blot is then corrected for any uneven DNA loading, to give the amplification levels shown in Table 3.

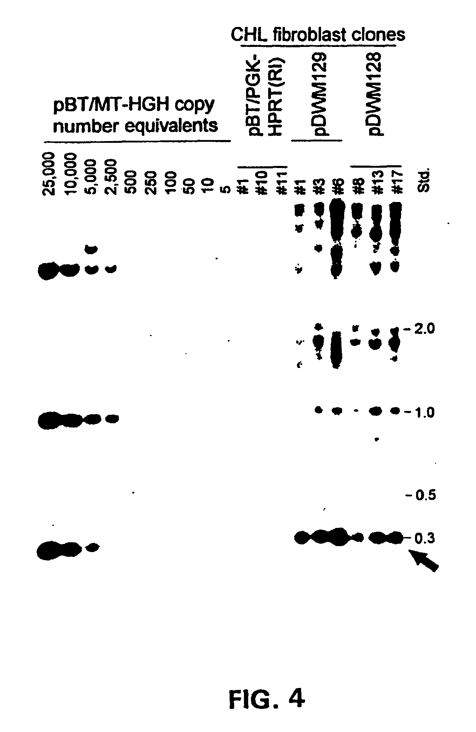

**Figure 4** Determination of MT-HGH gene copy number in HAT-resistant RJK88 cells following the one step gene amplification procedure. This was determined as described in the legend to Figure 3, except that the DNA was restricted with PstI/BglII, and probed with the HGH cDNA clone. The calibration curve was provided by restricting plasmid pBT/MT-HGH, with the indicated 270 bp internal PstI/BglII fragment being used to determine copy number. Note that the copy number determined from the blot is then corrected for any uneven DNA loading, to give the amplification levels shown in Table 3.

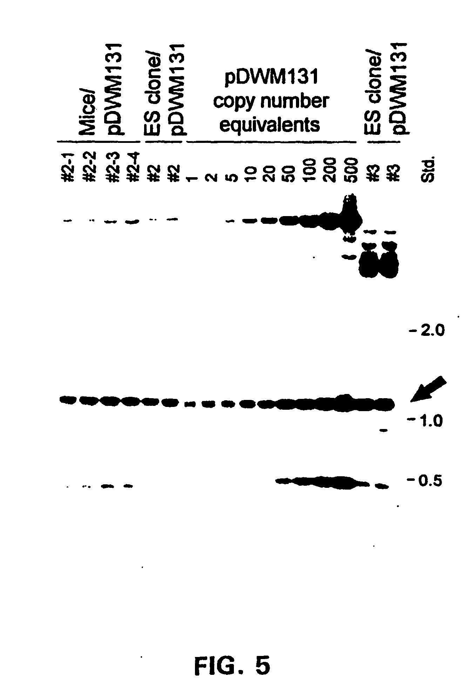

**Figure 5** Determination of HPRT minigene copy number in mouse embryonic stem cells and transgenic mice following the one-step gene amplification procedure. HM-1 cells were transformed with pDWM131. Genomic DNA (3 µg) from clones surviving the selection procedure was restricted with EcoRI/HindIII, electrophoresed and probed with the mouse HPRT cDNA clone pHPT5. In parallel, HM-1 DNA (3 µg) was spiked with varying amounts of pDWM131 plasmid DNA, corresponding to the indicated number of genomic equivalents, restricted with EcoRI/HindIII, electrophoresed on the same gel and probed to provide a calibration curve, against which the HPRT minigene copy number in the surviving HM-1 clones could be determined. This was done by comparing the intensity of the indicated 1.1 kb internal HindIII fragment. Two samples of each ES cell clone (pDWM131 # 2 and 3) are shown. The four transgenic mice are all progeny from a chimaera made with ES cell clone pDWM131#2.

### Example 1: Generation of Chinese hamster lung fibroblast cell lines with amplified copies of the human growth hormone gene, which secrete human growth hormone into the medium.

The one-step HPRT gene amplification system was tested in RJK88 cells - a non-reverting HPRT-deficient derivative of the V79 Chinese hamster lung fibroblast cell line (13). The human growth hormone (HGH) gene was chosen as the test gene for coamplification, because of its medical and commercial importance, and because convenient immunoradiometric assays exist to measure levels of growth hormone in cell culture medium. The HGH vector used was pBT/MT-HGH (Figure 2), which expresses a 670 bp HGH cDNA from a 770 bp fragment of the mouse metallothionein (MT)-I gene promoter, with the 3' untranslated region and polyadenylation signal provided by a 620 bp fragment from the HGH gene. The translation initiation region of the HGH cDNA was modified to give a strong Kozak consensus (14), but the vector was not modified in any other way to achieve high levels of HGH expression. Note, in particular, that the MT-I promoter fragment used is not a strong promoter. Thus, this vector provides a good test of the ability of the HPRT gene amplification system to express high levels of HGH.

One fully functional (pBT/PGK-HPRT (RI)) and two partially-disabled (pDWM128 and 129) HPRT minigenes were used in this experiment (see Figure 1). The HPRT minigenes were linearized with BamHI before being cotransformed into RJK88 cells by calcium phosphate-mediated transformation with pBT/MT-HGH, which had also been cut with BamHI. Each cotransformation used 20 µg of HPRT minigene DNA + 20 µg of HGH vector. HAT selection (4) was applied 24 hrs after transformation and surviving HAT-resistant (HAT^{R}) colonies were scored after 7-14 days (see Table 1). As expected, the two partially-disabled HPRT minigenes gave a lower frequency of HAT^{R} colonies than the fully functional minigene, pBT/PGK-HPRT (RI), with the most disabled minigene (pDWM128) giving the lowest transformation frequency of all.

Individual HAT^{R} colonies were picked from each of the three cotransformations and expanded in HAT medium. For each clone, the level of HGH extruded into the culture medium was determined by immunoradiometric assay. This was done by growing cultures to near confluence in 90mm dishes. The medium was then aspirated and replaced with 10ml of fresh medium. 24 hrs later the medium was collected and a sample taken for HGH assay. The cell monolayer was then lysed and a protein extract was made to determine the amount of cell protein/dish using the Lowry method. Thus, the concentration of HGH in the culture medium (in ng/ml) could be expressed /mg cell protein (see Table 2). The mean level of HGH secreted into the medium for clones cotransformed with the fully-functional HPRT minigene (pBT/PGK-HPRT (RI)) was 12.1 ng/ml/mg cell protein. The mean value was 7-fold higher for the partially-disabled minigene pDWM129, and 18-fold higher for the most disabled minigene pDWM128, where there should be selective pressure for the highest levels of HPRT gene amplification. Significantly, the four individual clones with the highest HGH levels (pDWM128# 5, 8, 13 and 31) all arose from the pDWM128 cotransformations. The highest HGH level obtained (pDWM128#8) was over 50-fold above the mean level found in clones cotransformed with the fully-functional HPRT minigene. The high HGH levels produced by some clones, cotransformed with partially-disabled HPRT minigenes, indicated that there had been coamplification of the MT-HGH gene.

The extent of HPRT minigene amplification and coamplification of the MT-HGH gene in selected HAT-resistant RJK88 clones was determined by Southern analysis. Genomic DNA, prepared from individual clones, was restricted to display an internal fragment from each of the two genes and then probed for either HPRT or MT-HGH sequences. The gene copy number was determined by comparing the hybridisation signal obtained from the internal gene fragment, against a calibration curve of a known number of genomic equivalents of plasmid DNA, spiked into RJK88 DNA and restricted in the same way as the genomic DNAs. The gene copy numbers are shown in Table 3 and selected Southern blots are shown in Figures 3 and 4. There was no evidence for HPRT minigene amplification, or coamplification of the MT-HGH gene, in the three clones assayed that were cotransformed with the fully-functional pBT/PGK-HPRT(RI) HPRT minigene. All clones assayed from cotransformations with the partially-disabled HPRT minigenes showed considerable HPRT minigene amplification and coamplification of the MT-HGH gene. In general, the highest levels of HPRT minigene amplification and MT-HGH gene coamplification were observed with the most disabled minigene pDWM128. HPRT minigene copy numbers ranged from 1,400 to > 10,000, while MT-HGH gene copy numbers were somewhat lower, ranging from 500 to 3,000. In general, the HPRT minigene and MT-HGH gene copy numbers in individual clones were very similar, suggesting the genes were amplified together as part of the same unit, but there were exceptions. There was not a good correlation between MT-HGH gene copy number and level of HGH production in the medium. This could arise from lack of expression of some of the amplified genes, or because some of the amplified units do not contain intact MT-HGH genes (only the internal 270 bp PstI-BglII fragment is being probed for in the Southern blots).

In conclusion, partially-disabled HPRT minigenes can be used in HPRT-deficient Chinese hamster lung fibroblasts to achieve one-step HPRT minigene amplification, with copy numbers in excess of 10,000, and coamplification of a human growth hormone gene, with copy numbers up to 3,000, and to produce high amounts of HGH in the culture medium.

### Example 2: Generation of mouse embryonic stem cell lines with amplified copies of a partially-disabled HPRT minigene

Electroporation of the HPRT-deficient mouse ES cell line, HM-1 (15), with the partially-disabled HPRT minigenes pDWM128 and 129 failed to generate any HAT-resistant survivors. Knowing that the requirements for HPRT expression were more fastidious in ES cells than in Chinese hamster lung fibroblasts ref (6), the experiment was repeated using the least disabled HPRT minigene, pDWM131, and a modified one-step selection procedure. 5 x 10⁶ HM-1 cells were electroporated with 25 µg of gel-purified HPRT minigene DNA, released from pDWM131 by restriction with SmaI/KpnI (see Figure 1). The electroporation conditions were 850V, 3 µFd, using our previously described procedure (16). 24 hours after electroporation, HAT selection was added. HAT selection was removed after a further 16 hrs and replaced with HT medium. HT medium is identical to HAT medium, except that it lacks Aminopterin and so is not selective for HPRT expression. The rationale was to reduce the stringency of the selective scheme and so make it easier for any clones containing amplified copies of the HPRT minigene to survive. A total of 8 colonies surviving the procedure were expanded in HAT medium for HPRT minigene copy number determination. In a parallel experiment, run under normal HAT selection conditions using the fully-functional pBT/PGK-HPRT (RI) minigene, a total of 350 colonies were obtained.

The pDWM131 minigene copy number in the HM-1 clones was determined as described in Example 1, except that an internal fragment of pDWM131 was used for comparison against the plasmid calibration. Clone pDWM131#1 contained around 10 minigene copies, clone #2 contained around 20 copies and clone #3 contained around 150 copies of the amplified minigene (see Figure 5). The remaining five of the eight clones examined showed no evidence for pDWM131 minigene amplification.

Thus, by using a less disabled HPRT minigene, the one-step gene amplification system can be used in embryonic stem cells to achieve HPRT minigene amplification, although the level of amplification observed was considerably lower than in Chinese hamster lung fibroblasts.

### Example 3: Generation of transgenic mice containing amplified copies of a partially-disabled HPRT minigene

Blastocyst injections were carried out with HM-1 clone pDWM131#2. Three chimaeras were produced, two male and one female. Test breeding of both male chimaeras resulted in transmission to progeny of the ES cell-derived coat colour markers. One chimaeric male was a 100% transmitter, passing the ES cell-derived coat colour markers to all 36 of its progeny. The second chimaeric male transmitted the ES cell-derived coat colour markers to 9/37 progeny. The female chimaera did not transmit the ES cell-derived coat colour markers. As expected, approximately half of the progeny carrying the ES cell coat colour markers were found to contain pDWM131 minigene sequences, by a PCR assay specific for the minigene carried out on DNA prepared from tail biopsies.

The PCR used primers from mouse HPRT cDNA sequence GenBank Acc No J00423. The primers were as follows.
exon 3 Forward (5')CTCGAGATGTCATGAAGGAGATGG
exon 9 Reverse (5')GCAGATGGCCACAGGACTAGAAC

The PCR comprised 35 cycles (94°C 1 min, 67°C 1 min, 72°C 1.5 min). 50µl reaction with 100ng template DNA, 300ng each primer, 2.5 units Taq DNA polymerase in 5 mM KC1/ 0.15 mM MgCl₂/ 1 mM Tris-HCl/ 0.045% Triton X-100/ 0.045% Tween 20/ 0.4 mM Na₂ EDTA/ 0.1 mM dNTPs. The resulting product size was 1.3 kb.

Southern analysis, on tail biopsy DNA from four of the transgenic animals containing pDWM131 minigene sequences, indicated that these mice contained the same number of copies (around 20) of the amplified pDWM131 minigene that were present in the starting ES cell clone, indicating that the amplified HPRT minigenes are present at a single chromosomal integration site (see Figure 5).

Thus, embryonic stem cells that have been through the one-step HPRT gene amplification system retain the potential for germ line transmission in chimaeric animals and transgenic mice with amplified HPRT minigenes, which are apparently stably inherited, can be produced.

### Example 4: Generation of transgenic mice expressing elevated levels of a human blood clotting regulatory protein in the milk

Next we investigated whether the one-step HPRT gene amplification system could be used to produce transgenic animals expressing high amounts of a valuable protein from a co-amplified nucleic acid sequence of interest. The gene chosen coded for a human regulatory protein involved in blood clotting. A construct (pBloodprotein) was available for this gene and comparative expression data from the milk of a range of conventional transgenic mice, containing variable numbers of randomly integrated transgenes was available. pBloodprotein comprises a human Protein C cDNA under the control of the sheep β-lactoglobulin gene promoter (Carver, A.S. *et al.* (1993) *Bio*/*Technology* **11**,1263-1270), with RNA processing signals provided by the 3' end of the sheep β-lactoglobulin gene. pBloodprotein had not been manipulated to maximise expression.

In two separate electroporations 10⁷ HM-1 cells were co-electroporated (900V, 3µFd) with 70 µg of pBloodprotein DNA (restricted with *Not*I to liberate the insert from the pBluescript vector) and 50µg of DNA from the disabled HPRT minigene, pDWM131 (cut with *Kpn*I/*Not*I to release the insert from the vector) and cells were plated at 2.5 x 10⁶ cells/90 mm dish. In both experiments HAT selection was added 24 hrs after electroporation, left on for 8 hrs and then replaced with HT medium. In the first experiment there were two further 8 hr periods of HAT selection, separated by growth in HT, in the two weeks between electroporation and colony picking. In the second experiment there were three further periods of HAT selection before colony picking. In both experiments picked colonies were rechallenged for 24 hrs in HAT medium before DNA was isolated from surviving colonies. 12 colonies were picked in the first experiment (clones # 1-12), of which 8 survived the final HAT challenge; 24 colonies were picked in the second experiment (clones # 101-124), of which 10 survived the final challenge. The HPRT minigene copy numbers were determined in the surviving clones as described previously and are shown in Table 4. HPRT minigene copy numbers ranged from 0-25 with, in general, higher numbers being obtained from the second experiment where the HAT selection was more severe. The human blood protein gene copy number was then determined for selected clones. Coamplification was observed, copy numbers ranging from 0-50, often with a good correlation between HPRT and pBloodprotein copy numbers in individual clones suggesting that the two sequences were part of the same amplified unit. Two of the clones with the highest amplified gene copy numbers (#10 and 117) were then subjected to continuous growth in HAT medium for 3 weeks to see if a further amplification could be achieved. The HPRT minigene and pBloodprotein copy numbers in clone #10 were unaltered, but in clone #117 the HPRT minigene copy number increased from 20-75 and the pBloodprotein copy number increased from 20-100.

To see if transgenic animals containing amplified copies of the human blood protein gene could be obtained three of the clones with the highest pBloodprotein copy numbers (#10,117,123) were then used for blastocyst injection. Two chimaeras were produced from a single injection session with clone #10, two from clone #123 and none from clone #117. Each of the chimaeras was test mated for germ-line transmission of the ES cell-derived coat colour by crossing with a Balb/C mouse. Both chimaeras from clone #10 transmitted the ES cell-derived coat colour (7% transmission for one chimaera, 19% for the other; see Table 4). One of the chimaeras from clone #123 was a low frequency transmitter (one pup only, which did not have the amplified sequences). As expected, approximately half the ES cell-derived offspring from the clone #10 chimaeras contained the amplified HPRT minigene and pBloodprotein sequences with copy number determinations in the first and subsequent generations indicating that the amplified sequences were stably inherited as a single unit with the same HPRT and pbloodprotein copy numbers as in the original ES cell clone.

To see if high levels of the human blood protein were present in the milk of animals containing amplified pBloodprotein sequences a male ES cell-derived offspring from one of the clone #10 chimaeras was mated and four of the resulting transgenic females (animals D-G) and three non-transgenic female littermate controls (animals A-C) were themselves mated and milk was collected manually at 7 days of lactation following oxytocin injection. The levels of the human blood protein in the milk were determined by an ELISA assay using a rabbit anti-human Protein C antibody available commercially (DAKO Ltd.). The ELISA assay data are shown in Table 5. The non-transgenic littermates all contained undetectable levels of the human protein while high levels were found in all four transgenic animals. The mean values ranged from 256-325 µg/ml. For comparison, the range of the human blood protein found in the milk of a series of independent conventional transgenic animals containing the identical pBloodprotein construct was 1-11 µg/ml.

Thus, the one-step HPRT gene amplification system can be used to produce ES cells with coamplified copies (~50) of a nucleic acid sequence of interest. Further amplification (~100 copies) can be achieved in some clones following extended growth in selective medium. ES cells with coamplified sequences retain their totipotency and transgenic animals can be produced containing the amplified sequences which are inherited stably. The animals can be used to produce large amounts of a valuable protein in the milk. In this example the levels of a human blood clotting regulatory protein were 30-fold higher than achieved in conventional transgenics.

**Table 1 Frequency of HAT-resistant colonies following cotransformation of HPRT minigenes and pBT/MT-HGH into RJK88 cells.**

| HPRT minigene | Frequency of HAT^{R} colonies^{a} |
|---|---|
| pBT/PGK-HPRT(RI) | 2.4 x 10⁻² |
| pDWM128 | 1.1 x 10⁻⁴ |
| pDWM129 | 1.8 x 10⁻³ |
| No DNA | 0 |

| | |
|---|---|
| Calcium phosphate cotransformadons were carried out on duplicate dishes of RJK88 cells (2 x 10⁵ cells/90mm dish for the partially-disabled HPRT minigenes, 2 x 10⁴ cells/90mm dish for pBT/PGKHPRT(RI)). 20µg of linearized HPRT minigene and 20µg of cut pBT/MT-HGH were used per cotransformation. ^{a} Mean frequency of HAT-resistant colonies/duplicate dishes of RJK88 cells plated. | |

**Table 2 Human growth hormone levels produced by individual HAT-resistant RJK88 clones cotransformed with pBT/MT-HGH and HPRT minigenes.**

| Clone | HGH (ng/ml medium/mg cell protein) |
|---|---|
| **pBT/PGK-HPRT (RI)** | |
| #1 | 26.3 |
| 2 | 0.1 |
| 3 | 38.5 |
| 4 | 0.3 |
| 5 | 0 |
| 6 | 0 |
| 7 | 0.5 |
| 8 | 0 |
| 9 | 44.8 |
| 10 | 15.6 |
| 11 | 1.4 |
| 12 | 17.1 |
| Mean (n=12) | 12.1 (± 16.5) |
| | |

| **pDWM 128** | |
|---|---|
| #1 | 13.9 |
| 3 | 81.6 |
| 5 | 448.8 |
| 6 | 177.4 |
| 7 | 30.1 |
| 8 | 657.3 |
| 10 | 57.5 |
| 12 | 51.2 |
| 13 | 498.0 |
| 14 | 117.6 |
| 16 | 20.9 |
| 17 | 229.7 |
| 18 | 71.3 |
| 21 | 265.1 |
| 31 | 555.6 |
| Mean (n=15) | 218.4 (± 217.5) |
| | |

| **pDWM 129** | |
|---|---|
| #1 | 272.4 |
| 3 | 194.4 |
| 4 | 48.6 |
| 5 | 28.4 |
| 6 | 23.4 |
| 7 | 2.6 |
| 9 | 21.2 |
| 10 | 5.2 |
| 11 | 123.3 |
| 12 | 210.6 |
| 13 | 21.9 |
| Mean (n=11) | 86.5 (± 96.9) |

**Table 3 Amplified HPRT minigene copy number and coamplified MT-HGH gene copy number in individual HAT-resistant RJK88 clones.**

| Clone | HPRT copy number | HGH copy number |
|---|---|---|
| **pBT/PGK-HPRT (RI)** | | |
| #1 | 1 | 1 |
| 10 | 1 | 1 |
| 11 | 2 | 1 |
| | | |

| **pDWM 128** | | |
|---|---|---|
| #8 | 2,100 | 2,100 |
| 10 | 10,000 | 1,000 |
| 13 | 2,800 | 2,800 |
| 17 | 3,000 | 3,000 |
| 21 | 5,000 | 1,100 |
| 31 | 5,000 | 2,700 |
| | | |

| **pDWM 129** | | |
|---|---|---|
| #1 | 1,700 | 1,700 |
| 3 | 1,400 | 2,600 |
| 5 | 5,000 | 500 |

**Table 4 Copy number of amplified HPRT minigene and coamplified nucleic acid sequence of interest in ES cells and transgenic animals.**

| ES clone # | HPRT copy # | Coamplified gene copy # | Blastocyst injection | Germ-line chimaeras | %Transmission | HPRT copies transmitted | Coamplified copies transmitted |
|---|---|---|---|---|---|---|---|
| 2 | 0 | 0 | -- | -- | -- | -- | -- |
| 3 | 10 | ND* | -- | -- | -- | -- | -- |
| 5 | 2 | ND | -- | -- | -- | -- | -- |
| 8 | 3 | ND | -- | -- | -- | -- | -- |
| 9 | 0 | ND | -- | -- | -- | -- | - |
| **10** | **20** | **50+** | **YES** | **2/2** | **7** | **20** | **50+** |
| | | | | | **19** | | |
| 11 | 0 | ND | -- | -- | -- | -- | -- |
| 12 | 0 | ND | -- | -- | -- | -- | -- |
| 103 | 10 | 2 | -- | -- | -- | -- | -- |
| 104 | 5 | ND | -- | -- | -- | -- | -- |
| 106 | 8 | ND | -- | -- | -- | -- | -- |
| 108 | 15 | 2 | -- | -- | -- | -- | -- |
| 109 | 20 | 0 | -- | -- | -- | -- | -- |
| **117** | **20** | **20** | **YES** | **0/0** | **--** | **--** | **--** |
| 118 | 20 | 5 | -- | -- | -- | -- | -- |
| 120 | 15 | 20 | -- | -- | -- | -- | -- |
| 122 | 7 | ND | -- | -- | -- | -- | -- |
| **123** | **25** | **50** | **YES** | **1/2** | **1** | **0** | **0** |
| *ND, not determined | | | | | | | |

**Table 5 Concentration of human blood clotting regulatory protein in the milk of transgenic animals containing amplified copies of the nucleic acid sequence of interest.**

| **Animal** | **Status** | **[Blood protein] (ug/ml)** |
|---|---|---|
| A | Non-transgenic littermate | 0 |
| B | Non-transgenic littermate | 0 |
| C | Non-transgenic littermate | 0 |
| D | Transgenic | 258 |
| E | Transgenic | 284 |
| F | Transgenic | 256 |
| G | Transgenic | 325 |
| The concentration in the milk of conventional transgenics containing the same construct was 1-11 ug/ml. | | |

### REFERENCES

1 . Kellems, R.E. 1993. Gene amplification in mammalian cells. Marcel Dekker Inc., New York, U.S.A.
2. International patent application W087/04462. 1987. Celltech Ltd and the University of Glasgow.
3. Gordon, J.W. and Isola, L.M. 1993. Spontaneous amplification of a foreign dihydrofolate reductase gene in transgenic mice. Transgene 1:77-90*.*
4. Littlefield, J.W. 1964. Selection of hybrids from matings of fibroblasts in vitro and their presumed recombinants. Science 145:709.
5. Melton, D.W. , Brennand, J., Ledbetter, D.H., Konecki, D.S., Chinault, A.C. and Caskey, C.T. 1982. Phenotypic reversion at the HPRT locus as a consequence of gene amplification. In Gene Amplification (R.T. Schimke, ed.), Cold Spring Harbor Laboratory, Cold Spring Harbor, New York, U.S.A., pp. 59-65.
6. Magin, T.M., McEwan, C., Milne, M., Pow, A.M., Selfridge, J. and Melton, D.W. 1992. A position and orientation dependent element in the first intron is required for expression of the mouse HPRT gene in embryonic stem cells. Gene 122:289-296.
7. Stacey, A., Schnieke, A., McWhir, J., Cooper, J., Colman, A. and Melton, D.W. 1994. Use of double-replacement gene targeting to replace the murine α-lactalbumin gene with its human counterpart in embryonic stem cells and mice. Mol. Cell. Biol. 14:1009-1016.
8. Moore, R.C., Redhead, N.J., Selfridge, J., Hope J., Manson, J.C. and Melton, D.W. 1995. Double replacement gene targeting for the production of a series of mouse strains with different prion protein gene alterations. Bio/Technology 13:999-1004.
9. Robertson, E.J. 1987. Teratomas and embryonic stem cells: a practical approach. IRL Press, Oxford, U.K.
10. Campbell, K.H.S., McWhir, J., Ritchie, W.A. and Wilmut, 1. 1996. Sheep cloned by nuclear transfer from a cultured cell line. Nature 380:64-66.
11. Wilmut, I., Schnieke, A.E., McWhir, J., Kind, A.J. and Campbell, K.H.S. 1997. Viable offspring derived from foetal and adult mammalian cells. Nature 385: 810-813.
12. Konecki, D.S., Brennand, J., Fuscoe, J.C., Caskey, C.T. and Chinault, A.C. 1982. Hypoxanthine phosphoribosyltransferase genes of mouse and Chinese hamster: construction and sequence analysis of cDNA recombinants. Nucl. Acids Res. 10:6763-6775.
13. Fuscoe, J.C., Fenwick, R.G., Jr., Ledbetter, D.H. and Caskey, C.T. 1983. Deletion and amplification of the HPRT locus in Chinese hamster cells. Mol. Cell. Biol. 3:1086-1096.
14. Kozak, M. 1986. Point mutations define a sequence flanking the AUG initiator codon that modulates translation by eukaryotic ribosomes. Cell 44:283-292.
15. Magin, T.M., McWhir, J. and Melton, D.W. 1992. A new mouse embryonic stem cell line with good germline contribution and gene targeting frequency. Nucl. Acids Res. 20:3795-3796.
16. Thompson, S., Clarke, A.R., Pow, A.M., Hooper, M.L. and Melton, D.W. 1989. Germ line transmission of a corrected HPRT gene produced by gene targeting in embryonic stem cells. Cell 56:313-321.
17. Jaenisch, R., Transgenic animals. Science 240, 1468-1474 (1988).
18. Page & Sydenham (1991), Bio/Technology 9:64.
19. Kaufman (1990), Meth. Enzymol. 185:487.
20. Kaufman (1990), Meth. Enzymol. 185:537.
21. Kaufman et al. (1987), EMBO J. 6:187.
22. Sambrook et al (1989), Molecular Cloning: A Laboratory Manual, pp.7.3-7.57 (Cold Spring Harbor Laboratory Press).
23. Sambrook et al (1989), Molecular Cloning: A Laboratory Manual, pp.18.1-18.88 (Cold Spring Harbor Laboratory Press).
24. Keown et al (1990), Meth. Enzymol. 185:527.
25. Lichtenstein et al (1987), Genetic Engineering 6:104 (Academic Press).
26. Clark AJ (1998) Biochem Soc Symp; 163:133-40.
27. Whitelaw CB et al. (1991) Transgenic Res; 1:3-13.

Prefferred embodiments of the application are as follows:
1. A method of making a transgenic non-human animal cell which is totipotent or totipotent for nuclear transfer and which cell comprises amplified copies of a nucleic acid sequence of interest, the method comprising subjecting a cell from a host cell population to a gene amplification protocol to produce a cell which retains its totipotency or totipotency for nuclear transfer and which comprises amplified copies of the nucleic acid sequence of interest.
2. A method according to claim 1 wherein the gene amplification protocol comprises transforming cells of the host cell population with the nucleic acid sequence of interest.
3. A method according to Claim 2 wherein the amplificadon protocol comprises:
   (a) cotransforming cells of the host cell population which are totipotent or which are totipotent for nuclear transfer with the nucleic acid sequence of interest and a partially-disabled selectable marker gene encoding a selectable gene product; and
   (b) culturing cells of the host cell population under conditions which permit selection of cells expressing the selectable gene product at significantly higher levels than cells with substantially no expression of the selectable gene product.
4. A method according to any one of the preceding claims wherein the gene amplification protocol comprises culturing cells under selective culture media such that only those cells expressing amplified copies of a selectable marker gene are capable of growth or survival in the selective culture medium.
5. A method according to claim 4 wherein the selectable marker gene encodes a product conferring drug resistance or a product which is capable of compensating for a metabolic or catabolic defect in the host cell.
6. A method according to any one of the preceding claims wherein the gene amplification protocol requires, or comprises, only one round of selection.
7. A method according to claim 6 wherein the amplification protocol comprises the steps of:
   (a) providing a plurality of partially-disabled selectable marker genes and wherein the selectable marker genes within said plurality possess differing degrees of expression disability;
   (b) selecting a gene from the plurality of partially-disabled selectable marker genes having regard to the nature of a host cell population to be transformed and the desired degree of gene amplification;
   (c) cotransforming a cell from the host cell population which is totipotent or which is totipotent for nuclear transfer with a nucleic acid sequence of interest and the selected partially-disabled selectable marker gene; and
   (d) culturing a cell of the host cell population under selective culture conditions, thereby providing selective pressure for amplification of the partially-disabled selectable marker gene and thereby achieving coamplification of the nucleic acid sequence of interest and thereby generating a cell which is totipotent or totipotent for nuclear transfer having amplified copies of the nucleic acid sequence of interest.
8. A method according to claim 3 or 7 wherein the partially-disabled selectable marker gene is a purine phosphoribosyltransferase.
9. A method according to claim 8 wherein the purine phosphoribosyltransferase is any one of the following: hypoxanthine phosphoribosyltransferase (HPRT), adenine phosphoribosyltransferase (APRT), guanine phosphoribosyltransferase (GPRT) or xanthine phosphoribosyltransferase (XPRT).
10. A method according to claim 9 wherein the partially-disabled selectable marker gene is HPRT.
11. A method according to any one of claims 3, 7, 8, 9 or 10 wherein prior to transforming cells of the host cell population with the selectable marker gene the cells did not comprise a functional or partially-disabled copy of the selectable marker gene.
12. A method according to any one of claims 3, 7, 8, 9, 10 or 11 wherein some or all of the partial-disablement of the selectable marker gene results from one or more of the following:
   (a) lack of a transcriptional control sequence normally present in the promoter region, or in an intron;
   (b) lack of an intron, or other mRNA processing signal;
   (c) a missense mutation, or a nonsense mutation;
   (d) insertion of an intron.
13. A method according to claim 3 or any one of claims 7 to 12 wherein the selectable marker gene is HPRT.
14. A method according to claim 13 wherein the selectable marker gene is a mammalian HPRT.
15. A method according to claim 14 wherein the selectable marker gene is a rodent HPRT, preferably murine HPRT.
16. A method according to claim 15 wherein the selectable marker gene is a murine HPRT minigene with one or more of the following characteristics:
   (a) a missense mutation, or a nonsense mutation in a HPRT coding region;
   (b) a truncated intron 1 (with the key control element present);
   (c) a truncated intron 2;
   (d) a truncated or absent intron 7 or 8.
17. A method according to claim 16 wherein the murine HPRT minigene has a missense mutation of Asp (GAT) 200 - Asn.
18. A method according to any one of the preceding claims wherein the host cell population comprises totipotent cells, preferably non-human embryonic stem (ES) cells.
19. A method according to any one of the preceding claims wherein the host cell population comprises cells totipotent for nuclear transfer, preferably cells derived from a non-human embryo, foetus or adult tissue.
20. A transgenic non-human animal cell which is totipotent or totipotent for nuclear transfer and which comprises amplified copies of a nucleic acid sequence of interest, which cell is obtainable by any one of the preceding claims.
21. A method of making a transgenic non-human animal which expresses multiple copies of a nucleic acid sequence of interest and which thereby produces a useful level of a product of interest, which method comprises employing a cell according to claim 20, or the genetic material thereof, to generate a transgenic non-human animal.
22. A method according to claim 21 wherein the generation of the transgenic non-human animal comprises injecting an ES cell, comprising amplified copies of the nucleic acid sequence of interest, into a host blastocyst or aggregating an ES cell with a host morula, and wherein a chimaeric embryo obtained thereby is implanted into a foster mother and is allowed to develop into a chimaeric non-human animal.
23. A method according to claim 22 wherein the method further comprises crossing the chimaeric non-human animal with another animal to obtain a transgenic non-human animal comprising coamplified copies of the nucleic acid sequence of interest in all its somatic cells.
24. A method according to claim 21 wherein the generation of the transgenic non-human animal comprises the step of transferring the nucleus of an animal cell having amplified copies of the nucleic acid sequence of interest into a recipient cell, preferably an enucleated oocyte.
25. A method according to any one of claims 21 to 24 wherein the transgenic non-human animal expresses substantial amounts of the product of interest, either constitutively or in a regulated manner, throughout the entire body or restricted to a particular tissue or body fluid.
26. A method according to claim 25 wherein the particular tissue is the mammary gland and the body fluid is milk.
27. A transgenic non-human animal which is totipotent or totipotent for nuclear transfer and which comprises amplified copies of a nucleic acid sequence of interest, which animal is obtainable by any one of the claims 21 to 26.
28. A method, non-human animal cell or non-human animal according to any one of the preceding claims wherein the non-human animal is a mammal.
29. A method, non-human animal cell or non-human animal according to claim 28 wherein the mammal is a placental mammal, preferably an ungulate, rodent or rabbit.
30. A method, non-human animal cell or non-human animal as claimed in according to any one of the preceding claims and substantially hereinbefore described.
31. A method, non-human animal cell or non-human animal as substantially hereinbefore described with reference to one or more of Examples 1 to 4.

## Claims

1. A method for amplifying a nucleic acid sequence of interest, comprising the steps of:
a) cotransforming a mammalian cell which is deficient for a purine phosphoribosyltransferase, with a nucleic acid of interest and a nucleic acid encoding a partially-disabled version of the purine phosphoribosyltransferase; and
b) culturing the mammalian cells in a selection medium, so as to permit selection of cells co-expressing and amplifying the partially-disabled purine phosphoribosyltransferase gene product and nucleic acid sequence of interest, at significantly higher levels than cells with substantially no expression of the purine phosphoribosyltransferase; wherein the method of amplification requires, or comprises, only one round of selection.

2. A method according to claim 1 wherein the purine phosphoribosyltransferase is any one of the following: hypoxanthine phosphoribosyltransferase (HPRT), adenine phosphoribosyltransferase (APRT), guanine phosphoribosyltransferase (GPT) or xanthine phosphoribosyltransferase (XPRT).

3. A method according to claim 2 wherein the partially-disabled selectable marker is HPRT.

4. The method according to any preceding claim wherein the amplification protocol comprises the steps of:
(a) providing a plurality of partially-disabled selectable marker genes and wherein the selectable marker genes within said plurality possess differing degrees of expression disability;
(b) selecting a gene from the plurality of partially-disabled selectable marker genes having regard to the nature of a host cell population to be transformed and the desired degree of gene amplification;
(c) contransforming a cell from the host cell population with a nucleic acid sequence of interest and the selected partially-disabled selectable marker gene; and
(d) culturing said transformed cells of the host cell population under selective culture conditions, thereby providing selective pressure for amplification of the partially-disabled selectable marker gene and thereby achieving coamplification of the nucleic acid sequence of interest and thereby generating a cell which has amplified copies of the nucleic acid sequence of interest.

5. The method according to any preceding claim wherein the selected cells allow production of large amounts of a product of interest.

6. A method according to any preceding claim wherein the partially disabled purine phosphoribosyltransferase is encoded by a murine HPRT mini-gene with one or more of the following characteristics:
(a) a missense mutation, or a nonsense mutation in a HPRT coding region;
(b) a truncated intron 1 (with the key control element present);
(c) a truncated intron 2;
(d) a truncated or absent intron 7 or 8.

7. The method according to claim 6 wherein the partially-disabled HPRT mini-gene contains an Asp(200)-Asn missense mutation, thereby encoding an HPRT protein with reduced catalytic activity and poor thermal stability.

8. Use of a vector comprising a partially-disabled HPRT mini-gene for coamplification in one round of selection, a gene of interest in a mammalian cell.

9. Use according to claim 8 wherein the gene of interest to be coamplified is present in the vector comprising said partially-disabled HPRT mini-gene.

10. Use of a partially expression-disabled HPRT mini-gene for the selection in HAT medium of cells containing up to thousands of copies of both said amplified HPRT mini-gene and a coamplified nucleic acid sequence of interest, wherein the nucleic acid sequence of interest is cotransformed, along with the partially-disabled HPRT mini-gene, into HPRT-deficient cultured mammalian cells.
